# EUROPEAN PATENT APPLICATION

(11) **EP 1 738 700 A1**
(43) Date of publication of application: **03.01.2007**
(21) Application number: 06013244.6
(22) Date of filing: 27.06.2006
(51) Int. Cl.: A61B 17/34, A61B 17/32

(54) **Thin bladed obturator with curved surfaces**

(30) Priority: 30.06.2005 US 170825
(71) Applicant: Tyco Healthcare Group LP, Norwalk, Connecticut 06856 (US)
(72) Inventor: Smith, Robert C., Cheshire, CT 06410 (US); Wenchell, Thomas, Durham, CT 06422 (US)
(74) Representative: HOFFMANN EITLE

(57) **Abstract**

A surgical obturator includes an obturator member having a distal end and a proximal end, and a blade member adjacent the distal end of the obturator member. The blade member includes first and second surfaces intersecting to define a peripheral cutting edge. At least one, preferably, both, of the first and second surfaces is curved. At least one, preferably, both, of the first and second surfaces is generally concave. The peripheral cutting edge defined by the first and second intersecting surfaces is substantially linear and obliquely arranged with respect to the longitudinal axis of the obturator member. The peripheral cutting edge may define an angle ranging from about 18° to about 22° with respect to the longitudinal axis. Alternatively, the peripheral cutting edge defined by the first and second surfaces is generally arcuate, and may be concave or convex in configuration. In accordance with one preferred embodiment, the blade member includes opposed pairs of intersecting first and second surfaces, which define opposed peripheral cutting edges. The opposed peripheral cutting edges extend to the penetrating end of the blade member. A hollow grinding technique is employed to form the curved surfaces and resulting cutting edges. Such technique provides significant benefits with respect to tissue resistance and drag.

## Description

### BACKGROUND

### 1. Technical Field

The present disclosure relates generally to a trocar assembly for use in minimally invasive surgical procedures, such as endoscopic or laparoscopic procedures. In particular, the present disclosure relates to a thin obturator blade for use with a trocar assembly and having cutting edges defined by curved surfaces of the blade.

### 2. Background of the Related Art

Minimally invasive procedures are continually increasing in number and variation. Forming a relatively small diameter temporary pathway to the surgical site is a key feature of most minimally invasive surgical procedures. The most common method of providing such a pathway is by inserting a trocar assembly through the skin. In many procedures, the trocar assembly is inserted into an insufflated body cavity of a patient. In such procedures, the trocar assemblies with seal mechanisms are utilized to provide the necessary pathway to the surgical site while minimizing leakage of insufflation gases.

Trocar assemblies typically include an obturator which is removably inserted through a cannula. The obturator may incorporate a penetrating end defining a general pyramidal or frusto-conical shape and having a sharpened or blunt point. In the alternative, the obturator may incorporate a thin bladed member such as the obturator blade disclosed in commonly assigned U.S. Patent Nos. 5,364,372, 5,545,150, 5,607,440, 5,797,443, 5,868,773 each to Danks, the contents of each patent being incorporated in their entirety by reference herein. Advantages of these thin bladed members include reduced penetration forces and smaller openings in the incision thereby reducing patient trauma and facilitating healing.

### SUMMARY

Accordingly, the present disclosure is directed to further improvements in bladed obturators. In one preferred embodiment, a surgical obturator includes an obturator member having a distal end and a proximal end, and a blade member adj acent the distal end of the obturator member. The blade member includes first and second surfaces intersecting to define a peripheral cutting edge. At least one, preferably, both, of the first and second surfaces is curved. At least one, preferably, both, of the first and second surfaces is generally concave. The peripheral cutting edge defined by the first and second intersecting surfaces is substantially linear and obliquely arranged with respect to the longitudinal axis of the obturator member. The peripheral cutting edge may define an angle ranging from about 18° to about 22° with respect to the longitudinal axis. Alternatively, the peripheral cutting edge defined by the first and second surfaces is generally arcuate, and may be concave or convex in configuration. In accordance with one preferred embodiment, the blade member includes opposed pairs of intersecting first and second surfaces, which define opposed peripheral cutting edges. The opposed peripheral cutting edges extend to the penetrating end of the blade member.

The surgical obturator may also include a protective shield coaxially mounted about the blade member. The protective shield and the blade member are adapted for relative longitudinal movement between a first armed position of the blade member and a second disarmed position of the blade member. Preferably, the protective shield is mounted for longitudinal movement relative to the obturator member. The protective shield may be normally biased to a position corresponding to the second disarmed position of the blade member.

In another embodiment, a surgical obturator includes an obturator member defining a longitudinal axis and having proximal and distal ends, and a generally planar blade member disposed adjacent the distal end of the obturator member. The blade member includes peripherally disposed opposed pairs of first and second generally concave surfaces intersecting to define opposed peripheral cutting edges. The peripheral cutting edges extend toward a penetrating end of the blade member. Each cutting edge extends in general oblique relation to the longitudinal axis. The cutting edges may be generally linear or arcuate. The surgical obturator also may include an obturator housing mounted adjacent the proximal end of the obturator member and a protective sleeve coaxially mounted about the obturator member. The protective sleeve is adapted for reciprocal longitudinal movement between an armed position of the blade member and a disarmed position of the blade member. The protective sleeve is biased to a position corresponding to the disarmed position of the blade member.

The curved or concave surfaces defining the cutting edges of the blade member are preferably formed via a hollow grinding technique. Such hollow grinding technique utilizes a rotating grinding wheel to grind an edge on the blade member perpendicular to the cutting edge. This yields a facet that when viewed in cross-section has a curvature resulting from the shape of the grinding wheel. The hollow grind and the cutting edges formed thereby provide substantial benefits in penetrating and passing through tissue particularly when compared to conventional flat grinding techniques which produce flat surfaces on an obturator blade, Specifically, the concave eonftgutation provided by the hollow ground provides sharper, more durable edges, thereby resulting in reduced penetration and drag through tissue.

### BRIEF DESCRIPTION OF THE DRAWINGS

The foregoing features of the present disclosure will become more readily apparent and will be better understood by referring to the following detailed description of preferred embodiments, which are described hereinbelow with reference to the drawings wherein:
FIG. 1 is a perspective view of an embodiment of a trocar assembly constructed in accordance with the present disclosure;
FIG. 2 is a perspective view with parts separated of the trocar assembly illustrating the cannula assembly and the obturator assembly in accordance with the embodiment of FIG.1;
FIG. 3 is a perspective view with parts separated of the obturator assembly in accordance with the embodiment of FIGS. 1-2;
FIG. 4 is a plan view of the protective shield of the obturator assembly in accordance with the embodiment of FIGS. 1-3;
FIG. 5 is a first plan view of the shield component of the protective shield in accordance with the embodiment of FIGS. 1-4;
FIG. 6 is a second plan view of the shield component of the protective shield in accordance with the embodiment of FIGS. 1-5;
FIG. 7 is an enlarged cross sectional view of the shield component of the protective shield taken along section line 7-7 of FIG, 6 in accordance with the embodiment of FIGS. 1-6;
FIG. 8 is a top plan view of the obturator blade of the obturator assembly in accordance with the embodiment of FIGS. 1-7;
FIGS. 8A-8B are top plan views of alternate embodiments of the obturator blade of the obturator assembly;
FIG. 9 is a side elevation view of the obturator blade illustrated in accordance with the embodiment of FIGS. 1-8;
FIG. 10 is a cross-sectional view of the obturator blade taken along section line 10-10 of FIG. 8 in accordance with the embodiment of FIGS. 1-9;
FIG. 11 is a cross-sectional view similar to the view of FIG. 10 illustrating an obturator blade of the prior art;
FIG. 12A-12E are cross-sectional views similar to the view of FIG. 10 of various embodiments of the obturator blade;
FIG. 13 is a side cross-sectional view of the obturator assembly in accordance with the embodiment of FIGS. 1-10;
FIG.14 is an enlarged cross-sectional view of the indicated area of detail of FIG. 13 in accordance with the embodiment of FIGS. 1-10 and FIG. 13;
FIG. 15 is a second enlarged cross-sectional view of the obturator housing of the obturator assembly in accordance with the embodiment of FIGS. 1-10 and FIGS. 13-14;
FIG.16 is a perspective view of the obturator shaft and the obturator blade in accordance with the embodiment of FIGS. 1-10 and FIGS. 13-15;
FIG. 17 is a perspective view of the indicator collar of the obturator housing mounted to the protective shield in accordance with the embodiment of FIGS. 1-10 and FIGS. 13-16;
FIG. 18 is a side cross-sectional view of the indicator collar and protective shield in accordance with the embodiment of FIGS. 1-10 and FIGS. 13-17;
FIG. 19 is a perspective view of the housing base of the obturator housing in accordance with the embodiment of FIGS. 1-10 and FIGS. 13-18;
FIG. 20 is a perspective view of the housing cover of the obturator housing in accordance with the embodiment of FIGS.1-10 and FIGS. 13-19;
FIGS. 21-22 are perspective views illustrating the components of the latch mechanism in accordance with the embodiment of FIGS. 1-10 and FIGS. 13-20;
FIG. 23 is a side cross-sectional view of the trocar assembly illustrating the obturator assembly mounted relative to the cannula assembly and the latch member in an actuated position in accordance with the embodiment of FIGS. 1-10 and FIGS, 13-22;
FIG. 24 is an enlarged side cross-sectional view illustrating the relationship of the components of the latch member in an actuated position in accordance with the embodiment of FIGS. 1-10 and FIGS. 13-23;
FIG. 25 is a view similar to the view of FIG. 23 illustrating the protective shield of the obturator assembly in a retracted position in accordance with the embodiment of FIGS. 1-10 and FIGS. 13-24; and
FIG. 26 is a view similar to the view of FIG. 24 illustrating the relationship of the components of the latch member when the protective shield is in the retracted position in accordance with the embodiment of FIGS. 1-10 and FIGS. 13-25.

### DETAILED DESCRIPTION OF PREFERRED EMBODIMENTS

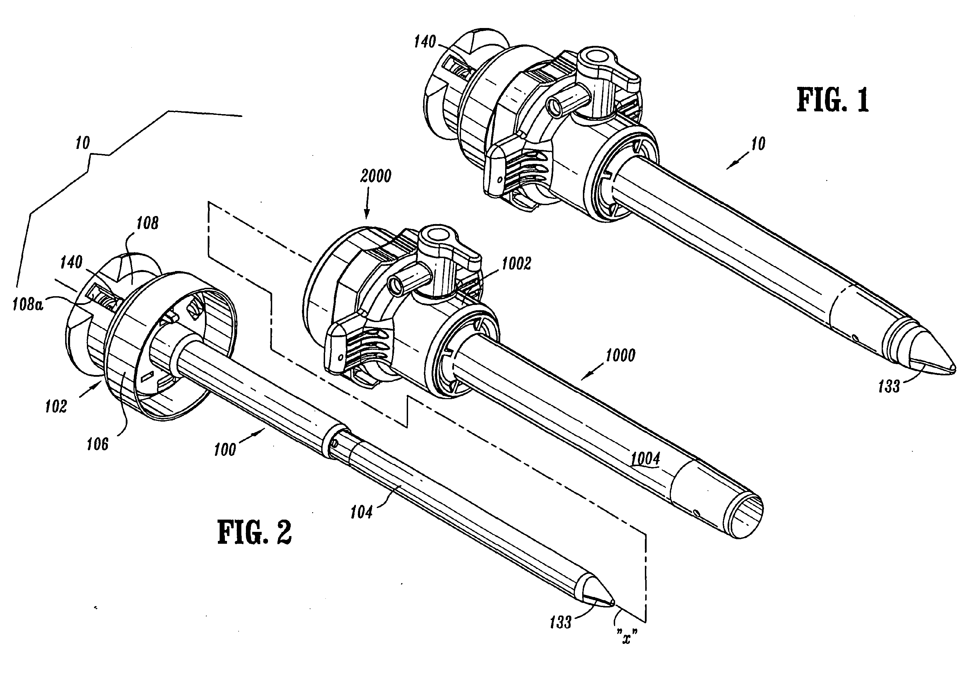

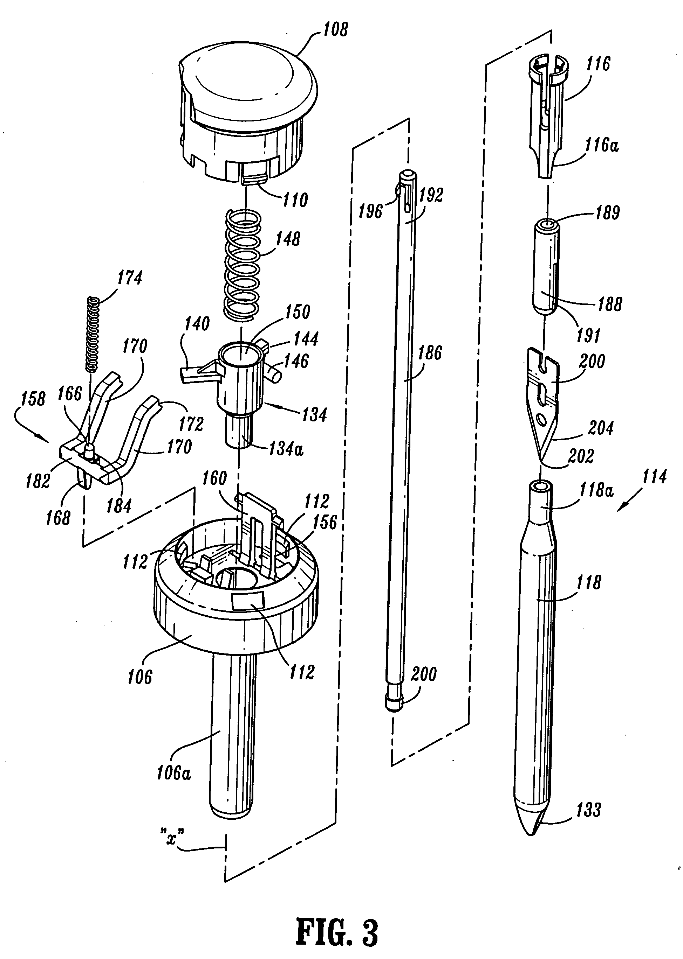

Referring now in detail to the drawing figures, in which like references numerals identify similar or identical elements, there is illustrated, in FIGS. 1 and 2, a trocar assembly constructed in accordance with a preferred embodiment of the present disclosure and designated generally by reference numeral 10. Trocar assembly 10 is particularly adapted for use in minimally invasive surgical procedures such as endoscopic or laparoscopic procedures. Generally, trocar assembly 10 includes two principal subassemblies, namely, obturator assembly 100 and cannula assembly 1000.

Cannula assembly 1000 may be any cannula assembly suitable for use in a laparoscopic surgical procedure. In one preferred embodiment, cannula assembly 1000 includes cannula housing 1002 and cannula sleeve 1004 extending from the cannula housing 1002. Either or both cannula housing 1002 and cannula sleeve 1004 may be transparent in part or in whole and are fabricated from biocompatible metal or polymeric material. Cannula assembly 1000 may include an internal seal such as a duck-bill valve or other zero closure valve adapted to close in the absence of a surgical instrument to prevent passage of insufflation gases through the cannula assembly 1000.

Trocar assembly 10 may also include a seal assembly 2000 which is preferably releasably mounted to cannula housing 1002. Means for releasably connecting seal assembly 2000 to cannula housing 1002 may include a bayonet coupling, threaded connection, latch, friction fit, tongue and groove arrangements, snap-fit, etc, Seal assembly 2000 may include seal housing 2002 and at least one internal seal which is adapted to form a fluid tight seal about an instrument inserted through the seal assembly 2000. One suitable seal may be the fabric seal disclosed in commonly assigned U.S. Patent Application Serial No.10/165,133, filed June 6, 2002, the entire contents of which are incorporated herein by reference. The seal disclosed in the '133 application maybe a flat septum seal having a first layer of resilient material and a second fabric layer juxtaposed relative to the first layer. Further details of the seal may be ascertained by reference to the '133 application. Seal assembly 2000 may or may not be a component of cannula assembly 1000, For example, the seal assemblymaybe a separate, removable assembly. In the alternative, the seal assembly may comprise an integral part of the cannula assembly 1000 and not be removable.

With reference now to FIGS. 2-3, obturator assembly 100 includes obturator housing 102 and obturator shaft 104 extending distally from the housing 102. Obturator shaft 104 defines obturator axis "x", Obturator housing 102 includes housing base 106 and housing cover 108. Once the appropriate components are positioned therewithin (as described below), housing base 106 may be attached to housing cover 108 by engaging mating surfaces, for example, by resilient latches 110 of cover 108 interlocking with correspondingly dimensioned latch openings 112 of housing base 106. Preferably, to uniformly connect base 106 and cover 108 at least three corresponding latches 110 and openings 112 are spaced evenly around the circumference of the cover 108 and the base 108, respectively. Preferably, obturator housing 102 is configured and dimensioned to functionally cooperate with cannula that range in size, e.g,, from about 5 mm to about 15 mm in diameter.

Obturator assembly 100 further includes protective shield 114. Protective shield 114 includes shield extension 116 and shield member 118 operatively connected to each other to define an outer member of obturator assembly 100. Any means for connecting shield extension 116 and shield member 118 are envisioned including, e.g., a bayonet coupling, snap fit, tongue and groove arrangement, interference fit. Alternatively, shield extension 116 and shield member 118 may be a single component, monolithically formed during manufacture. In a further alternative, shield extension 116 could be eliminated.

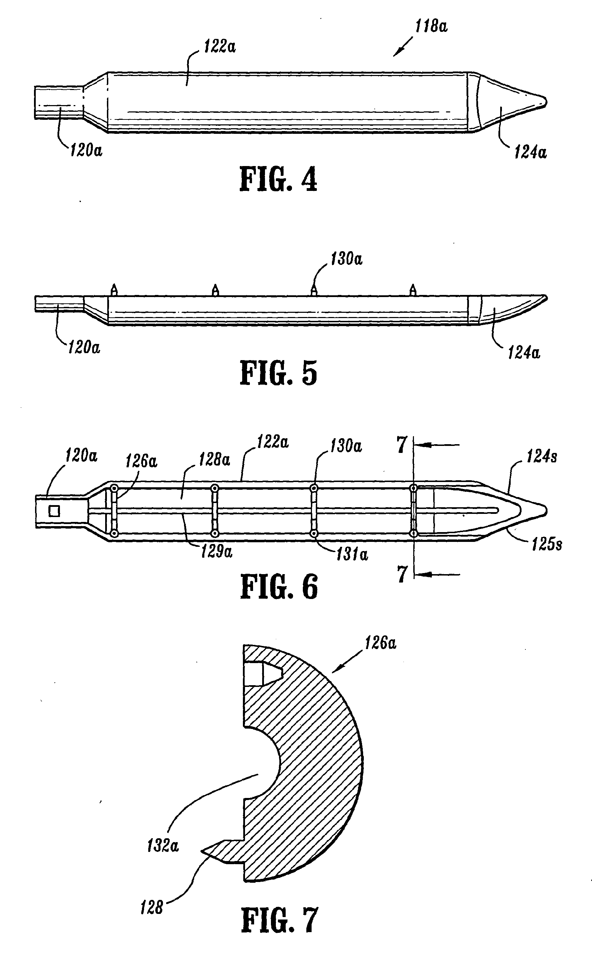

With reference to FIGS. 4-7, in conjunction with FIGS. 2-3, shield member 118 includes first and second shield half-sections 118a, 118b. First and second shield half-sections 118a are identically shaped. Each half-section 118a includes a reduced collar extension 120a, a semicircular main portion 122a, and a distal blade guard section 124a. Reduced collar extensions 120a receive the distal end 116a of shield extension 116 to facilitate connection of the two components. Each shield half-section 118a further includes transverse walls 126a equidistally spaced along the longitudinal axis. Each transverse wall 126a includes an upstanding notch 128 adjacent one end of the wall 126a and a substantially cylindrical recess 130 adjacent the other end of the transverse wall 116a. In the assembled condition ofhalf sections 118a, notches 128 are received within corresponding recesses 130 thereby maintaining proper alignment and symmetry of the half sections 118a. It is envisioned that notches 128 and recesses 130 may be dimensioned to form a friction or interference fit between the components or structured to form a snap fit arrangement. It is further noted that adhesives, cements, etc. may be used to fixedly secure the half sections 118a to each other. Each transverse wall 116 further defines semicircular recess 132a which, in the assembled condition of half sections 118a, forms a circular passage or recess through shield member 118.

Blade guard sections 124a define a general tapered configuration when assembled. The tapered configuration may be in the general shape of a cone or conic section. Alternatively, the tapered configuration may be in the general shape of a dolphin-nose having a pair of diametrically opposed surfaces 124s which are slightly concave in appearance (see FIG. 6). Such arrangement is disclosed in the aforementioned commonly assigned U.S. Patent Application Serial No. 11/103,892, filed April 12, 2005, the contents of which are incorporated by reference. Blade guard sections 124a further define a blade receiving slot 133 when assembled (FIGS. 1-3).

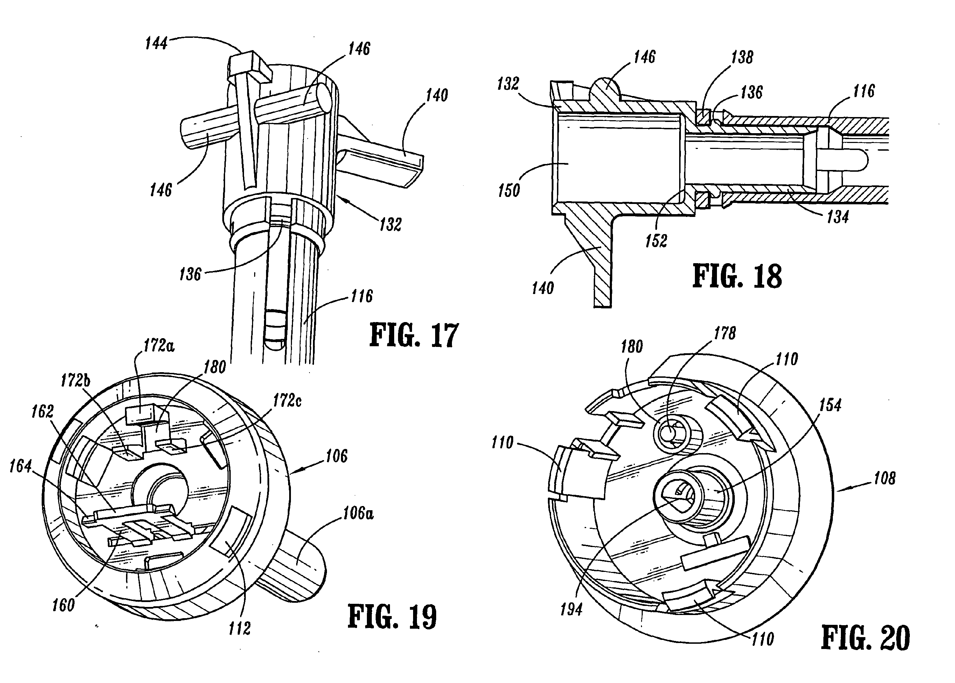

Referring now to FIGS. 3 and 13-18, obturator assembly 100 further includes indicator collar 134 which is secured within the proximal end of protective shield 116. In one preferred arrangement, indicator collar 134 defines distal collar extension 134a. Collar extension 134a includes annular rim 136 on its outer surface. Annular rim 136 is contained within protective shield 116 through its engagement with radial tabs 138 of protective shield 116 as best shown in FIGS. 17 and 18.

Indicator collar 134 further includes a shield position indicator, such as indicator flag 140, extending transversely relative to the indicator collar 134. Indicator flag 140 is visible from the exterior of obturator housing 102 as it extends through groove 108a of housing cover 108 (see also FIGS. 2 and 17). Preferably, indicator flag 140 is colored to contrast sharply with the surrounding housing components. For example, indicator flag 140 may be red if the surrounding housing components are white or light colored. Indicator collar 134 further includes collar ledge 144 and a pair of posts 146 formed below the ledge 144 and extending radially outwardly from the ledge 144. Collar ledge 144 serves to releasably lock protective shield 114 in a distal position with respect to blade 190.

Indicator flag 140 and protective shield 114 are spring biased in the distal direction by coil spring 148. In particular, coil spring 148 is received within internal bore 150 of indicator collar 134 and engages internal shelf 152 (see FIG. 18) of the indicator collar 134. The proximal end of coil spring 138 is coaxially mounted about spring mount 154 (see FIG. 20) depending from the interior surface of housing cover 108,

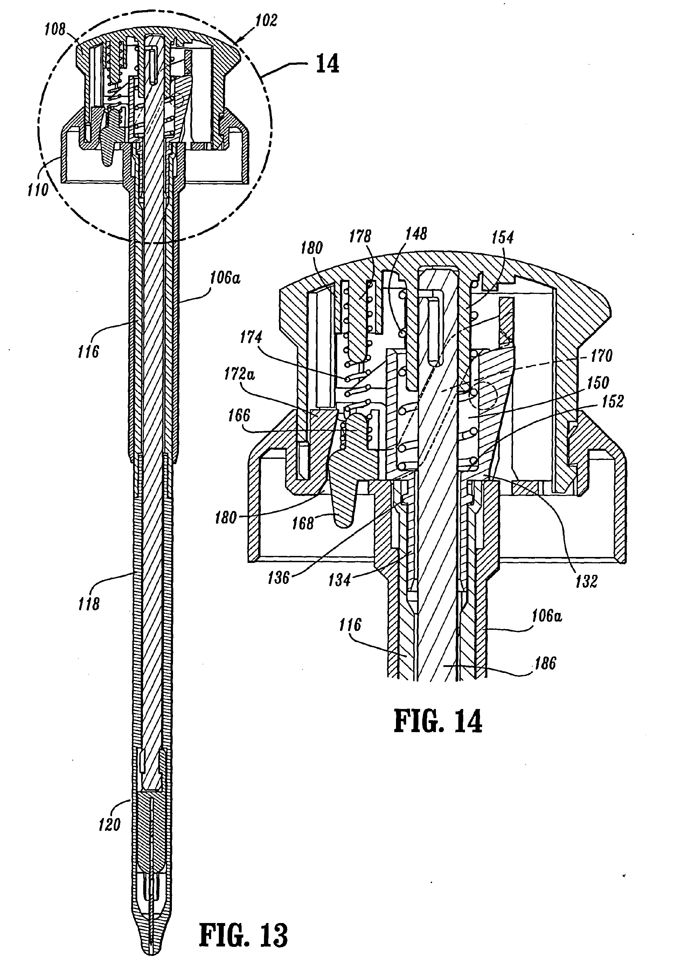

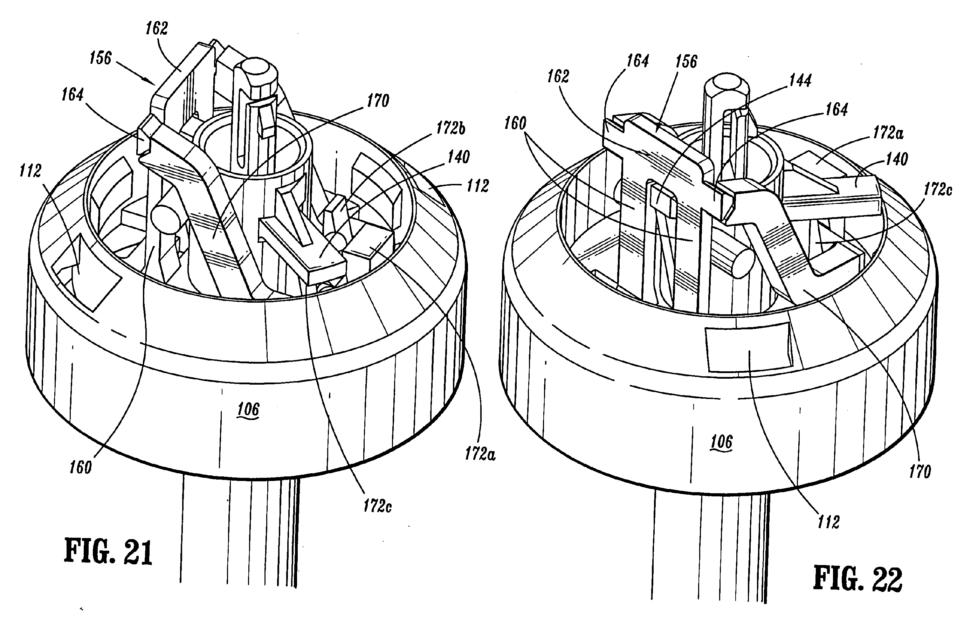

Referring now to FIGS. 19-22, in conjunction with FIG. 3, obturator assembly 100 includes a latching mechanism disposed within obturator housing 102 to prevent proximal movement of protective shield 114 until such time as the obturator assembly 100 is mounted to cannula assembly 1000 and the surgeon is prepared to begin trocar entry. Latching mechanism includes latch member 156, and release member such as slider 158, as best seen in FIG. 3. Latch member 156 has two vertical legs 160 connected by web 162. A pair of biasing posts 164 extends outwardly, one for each side of latch member 156. Collar ledge 144 of indicator collar 134 is engaged and secured by web 162 of latch member 156 when in an initial position of the latch member 156 as depicted in FIGS. 21-22. In the initial position of latch member 156, protective shield 114 is retained in a first extended position shown in FIG. 14. Latch member 156 is preferably molded as part of housing base 106 in cantilever fashion. However, latch member 156 may be formed as a separate element and secured to base 106 by suitable known techniques.

Slider 158 includes post 166 disposed at its lower end, arming button 168 extending distally from the distal face of slider 158 and a pair of slider legs 170 which terminate in crooks 172. Crooks 172 defined in slider legs 170 are configured and dimensioned to engage posts 164 of latch member 156, as shown in FIGS. 21 and 22. Slider 158 is distally biased by slider spring 174 which is maintained in axial alignment by slider post 166 of slider 158. The proximal end of slider spring 174 bears against the inner surface of housing cover 108 and is maintained in position between proximal post 178 and cylindrical base 180 formed in base 106, (see FIGS. 19 and 20). The distal biasing of slider 158 causes arming button 168 to project through opening 180 formed in housing base 106. The lower end or transverse leg 182 of slider 158 resides with mounting posts 172 a-c of housing base 106 with post base 184 of slider 158 residing within mounting posts 172b, 172c. (See FIG. 19). Mounting of obturator assembly 100 relative to cannula assembly 112 causes slider 158 to translate or rotate generally vertically in a generally proximal direction as will be described further hereinbelow.

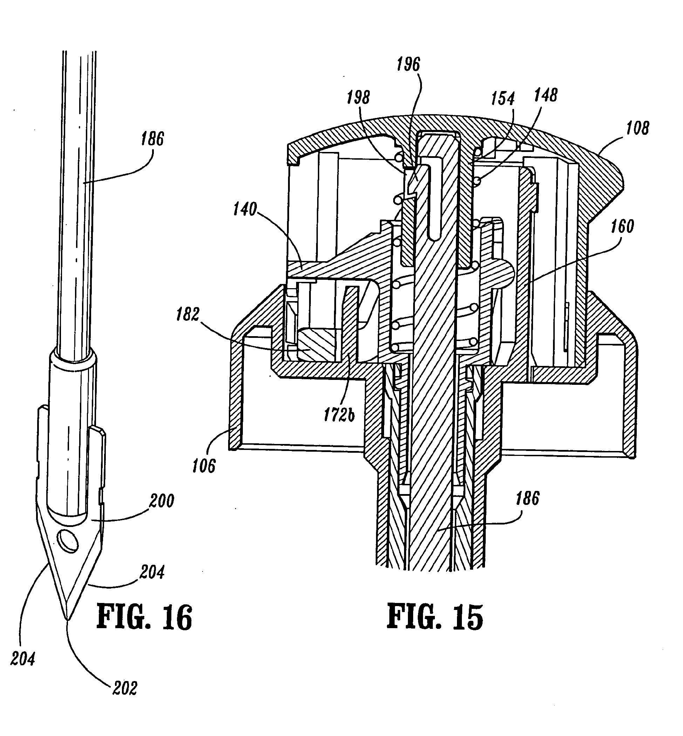

With reference to FIGS. 13-15, in conjunction with FIG. 3, the components of obturator shaft 104 will be discussed. Obturator shaft 104 includes obturator rod 186, blade mount 188 and obturator blade 200. Obturator rod 186 defines proximal end 192 having a general semi-circular cross-section and being received within correspondingly dimensioned opening 194 of spring mount 154 of housing cover 108 (see FIG. 20). The proximal end 192 further defines latch 196 which is received within side opening 198 of spring mount 154 in snap relation therewith to secure the two components (see FIG. 15).

With reference now to FIG. 16, in conjunction with FIGS. 13-15 and FIG. 3, the distal end of obturator rod 186 defines collar 190. Blade mount 188 includes mounting recess 189 which is dimensioned to receive collar 190 of obturator rod 186. Blade mount 188 further includes knife slot 191 which accommodates obturator blade 200. Obturator blade 200 may be secured within knife slot 191 by conventional means including adhesives, cements, etc.

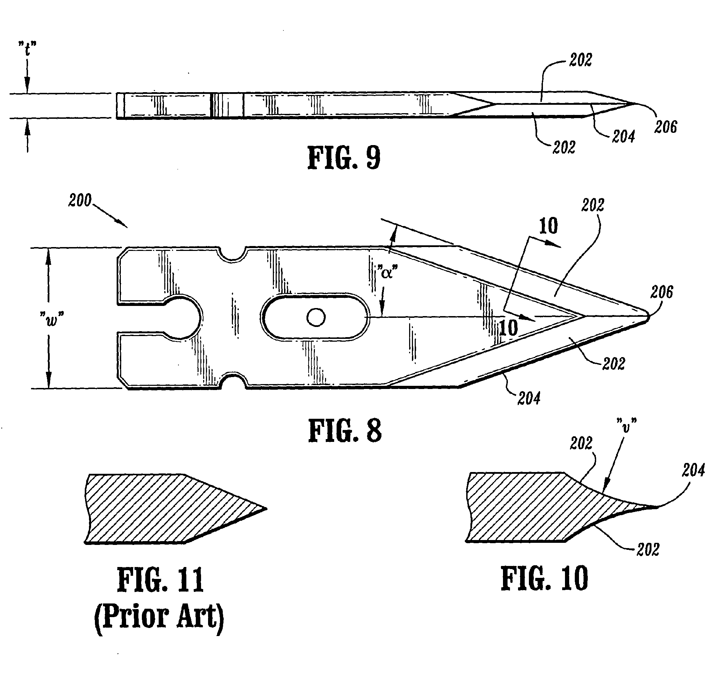

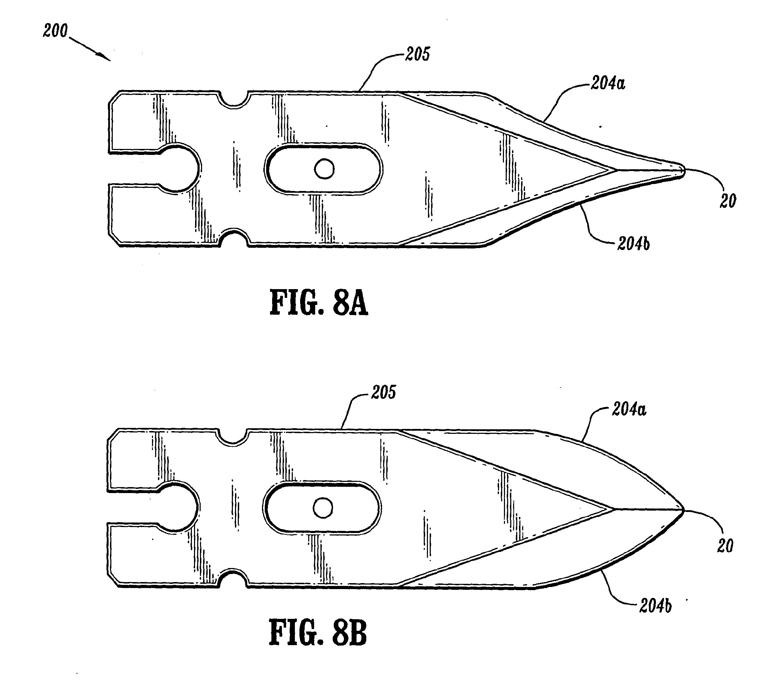

Referring now to FIGS. 8-11, in conjunction with FIG. 3, obturator blade 200 will now be discussed. Obturator blade 200 is substantially thin defining a thickness "t" substantially less than a width "w". Obturator blade 200 is generally blade-like in appearance and may be a planar blade or the like. Obturator blade 200 includes pairs of opposed curved surfaces 202 formed into the blade 200 and defining peripheral cutting edges 204 along the lines of intersection of the curved surfaces 202. Curved surfaces 202 are generally concave in configuration defining a radius of curvature "v" ranging from about .05 inches to about 3.55 inches. Peripheral cutting edges 204 extend at an angle "α" relative to longitudinal axis. Angle "α" may range from about 18° to about 22°, preferably, about 20°. Cutting edges 204 terminate adjacent penetrating point 206. Penetrating point 206 may be sharp or blunt.

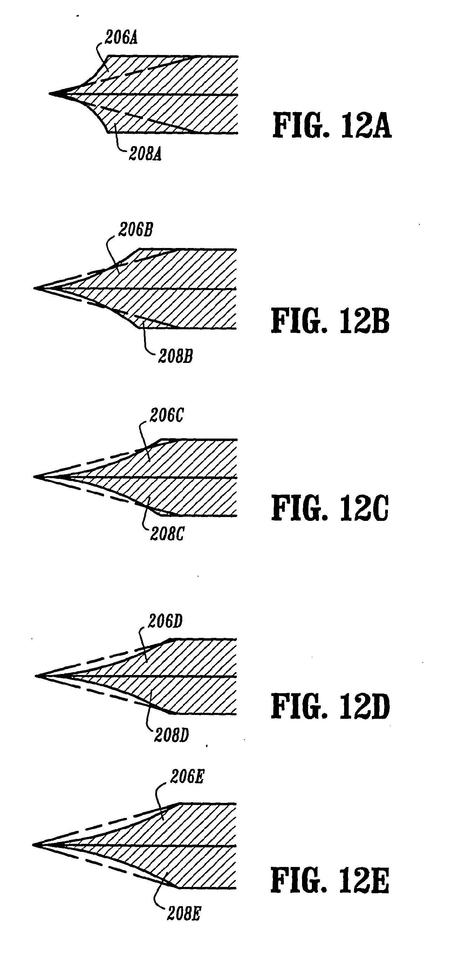

In a preferred embodiment, curved surfaces 202 may be made through conventional means, including, for example, coining, grinding, etc. In one preferred method of manufacture, curved surfaces 202 are formed via a hollow grinding technique. Such hollow grinding technique utilizes a rotating grinding wheel which is used to grind an edge on a blade perpendicular to the cutting edge. This yields a facet that when viewed in cross-section has a curvature resulting from the shape of the grinding wheel. The hollow grind and the tapered edges formed thereby provide substantial benefits in penetrating and passing through tissue particularly when compared to conventional flat grinding techniques which produce flat surfaces on an obturator blade (See the prior art obturator blade of FIG. 11). Specifically, the concave configuration provided by the hollow ground provides sharper edges, thereby resulting in reduced penetration and drag through tissue. FIGS. 12A-12E illustrates surfaces 206, 208 having a variety of radius of curvatures. (In FIGS. 12A-12E, linear cutting edges of the conventional or prior art blade of FIG.11 are shown in phantom lines.) As indicated hereinabove, the radii of curvature of curved surfaces may vary in accordance with the desired objective and characteristic of the blade. For example, FIG. 12A illustrates surfaces 206A, 208A having a 0,020 radius of curvature. FIG. 12B illustrates surfaces 206B, 208B having a radius of about .050 inches curvature. FIG. 12C illustrates surfaces 206C, 208C having a radius of about .070 inches curvature. FIG. 12D illustrates surfaces 206D, 208D having a radius of curvature of .080 inches. FIG. 12E illustrates surfaces 2061;, 208E having a radius of curvature of .090 inches. It is also envisioned that cutting edges 204 formed by curved surfaces 202 may have a concave or convex appearance instead of the linear edge shown in FIG. 8. The concave and convex arrangements of cutting edges 204a, 204b are shown in FIGS. 8A and 8B, respectively.

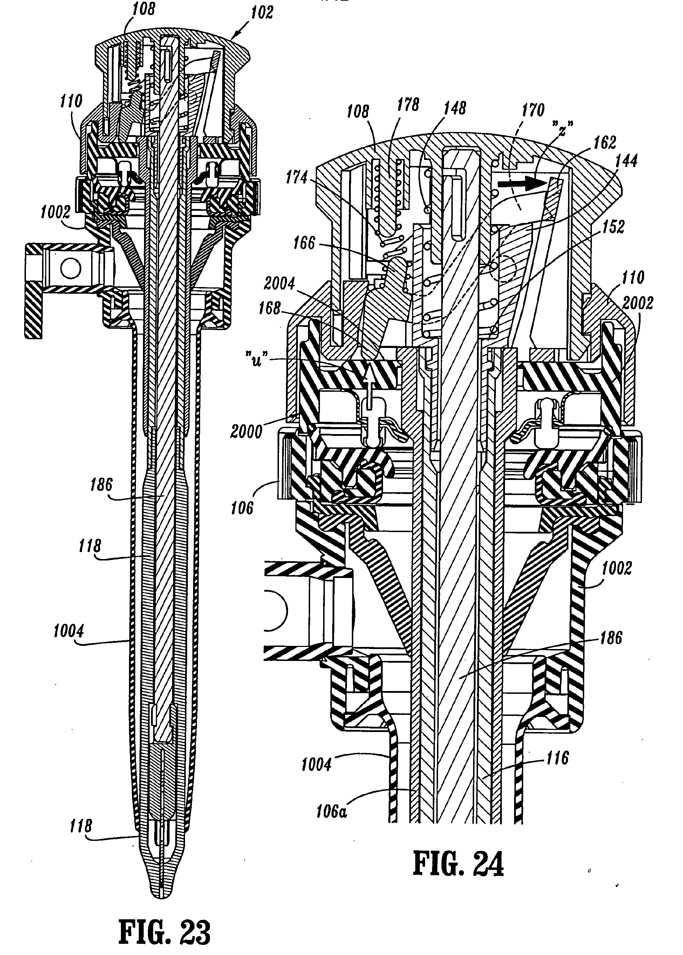

With reference now to FIGS, 23-24, a method of use and operation of trocar assembly 10 will be discussed. Obturator assembly 100 is inserted within cannula assembly 1000 and advanced to where obturator housing 102 is approximated with seal housing 2002 of the seal assembly 2000. Seal assembly 2000 may comprise a separate part or may be a component of cannula assembly 1000. Seal housing 2002 and housing base 106 of obturator housing 102 may be appropriately dimensioned to form a friction fit or may be coupled to each other by conventional means including bayonet coupling, tongue-groove, etc. Approximating the obturator housing 102 and the seal housing 2002 releases the blade guard 120 from a locked condition, actuating the trocar assembly. With the obturator housing 102 and seal housing 2002 approximated, arming button 168 of slider 158 engages surface 2004 of seal housing 2002 and is forced upwardly (depicted by directional arrow "u") from the position depicted in FIG. 14 to the position depicted in FIGS. 23-24. During this movement, slider 158 pivots or angulates whereby legs 170 of slider 158 push latch member 156 in a radial outward direction (depicted by directional arrow "z") such that web portion 162 of latch member 156 is out of axial alignment with ledge 144 of indicator collar 134. In this position, indicator collar 134 and protective shield 114 are free to axially move.

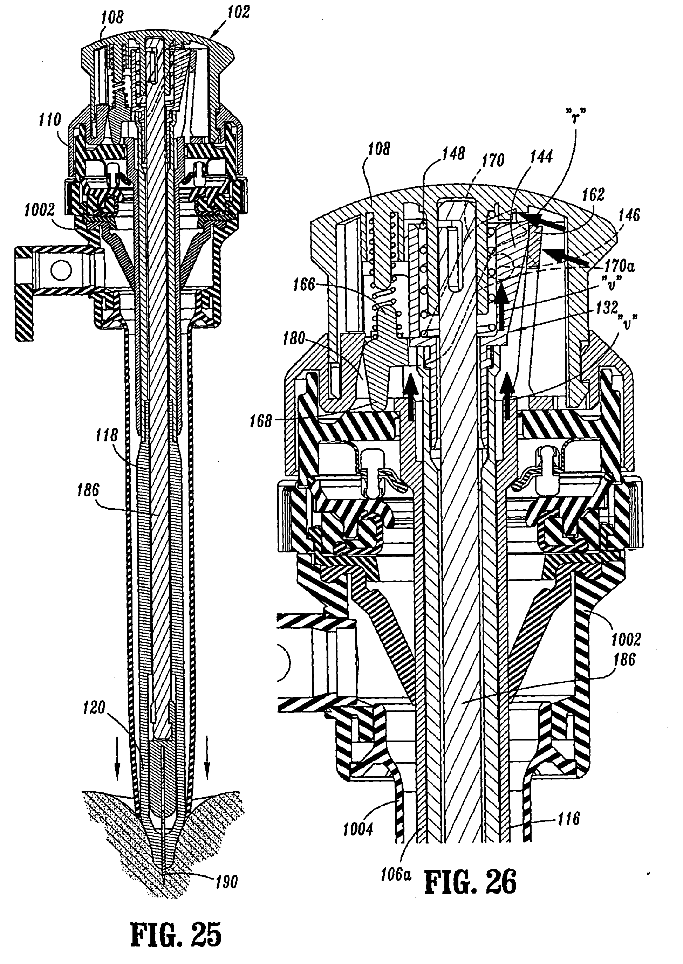

Referring now to FIGS. 25-26, the surgeon begins to insert trocar assembly 10 through the body wall of the patient. Shield assembly 118 contacts the tissue and is driven upwardly to cause protective shield 114 and indicator collar 134 to move proximally (depicted by directional arrow "v") against the bias of coil spring 148. Such movement exposes obturator blade 190 to incise the tissue. This armed condition of obturator assembly 100 is visually verified by the proximal location of indicator flag 140 of indicator collar 134. In addition, proximal movement of indicator collar 134 causes posts 146 of the indicator collar 134 to ride along outer surfaces 170a of legs 170 of slider 158 to thereby move the slider 158 at least radially inwardly and upwardly (as shown by the directional arrows "r") in a general aligned position relative to the obturator axis "x". FIG. 26 illustrates this actuated position of latch member 156. With obturator blade 200 exposed, the surgeon may apply a distally-directed force to obturator assembly 100 to cause penetration through the tissue.

Once obturator blade 200 and the guard portion of shield assembly 118 passes through the body wall of the patient, protective shield 114 moves distally to cover obturator blade 200. Ledge 144 of indicator collar 134 also moves into engagement with web portion 162 of latch member 156. In particular, indicator collar 134 and protective shield 114 are driven distally under the influence of coil spring 148. Concurrently with this movement, slider 158, which is aligned relative to axis "x", is driven distally under the influence of coil spring 174. In the respective positions of indicator collar 134 and slider 158 depicted in FIGS. 13 and 14, collar ledge 144 of indicator collar 134 securely engages web 162 of latch member 156 to secure protective shield 114 in its extended position. The obturator assembly 100 is removed from cannula assembly 1000 and surgery is performed with instruments inserted through cannula assembly 1000.

Except where noted otherwise, the materials utilized in the components of the presently disclosed trocar assembly generally include materials such as, for example, ABS, polycarbonate, stainless steel, titanium and any other suitable biocompatible metals and/or polymeric materials. A preferred ABS material is CYCOLAC which is available from General Electric. A preferred polycarbonate material is also available from General Electric under the trademark LEXAN. An alternative polycarbonate material which may be utilized is CALIBRE polycarbonate available from Dow Chemical Company. The polycarbonate materials may be partially glass filled for added strength.

It will be understood that various modifications and changes in form and detail may be made to the embodiments of the present disclosure without departing from the spirit and scope of the invention. Therefore, the above description should not be construed as limiting the invention but merely as exemplifications of preferred embodiments thereof. Those skilled in the art will envision other modifications within the scope and spirit of the present invention as defined by the claims appended hereto. Having thus described the invention with the details and particularity required by the patent laws, what is claimed and desired protected is set forth in the appended claims.

## Claims

1. A surgical obturator, which comprises:
an obturator member having a distal end and a proximal end; and
a blade member adjacent the distal end of the obturator member, the blade member including first and second surfaces intersecting to define a peripheral cutting edge, at least one of the first and second surfaces being curved.

2. The surgical obturator according to claim 1, wherein the first and second surfaces are each curved.

3. The surgical obturator according to claim 1 or 2, wherein the at least one surface is generally concave.

4. The surgical obturator according to claim 1, 2 or 3, wherein the at least one surface is formed via a hollow grinding process.

5. The surgical obturator according to any one of the preceding claims, wherein the peripheral cutting edge is substantially linear.

6. The surgical obturator according to claim 5, wherein the cutting edge is obliquely arranged with respect to the longitudinal axis of the obturator member.

7. The surgical obturator according to claim 6, wherein the cutting edge defines an angle in a range of from 18° to 22° with respect to the longitudinal axis.

8. The surgical obturator according to any one of claims 1 to 4, wherein the peripheral cutting edge is generally arcuate.

9. The surgical obturator according to claim 8, wherein the peripheral cutting edges is generally concave.

10. The surgical obturator according to any one of the preceding claims, wherein the blade member includes opposed pairs of intersecting first and second surfaces to define opposed peripheral cutting edges.

11. The surgical obturator according to any one of the preceding claims, wherein the blade member defines a penetrating end.

12. The surgical obturator according to claim 11, as dependent on claim 10, wherein the opposed peripheral cutting edges extend to the penetrating end of the blade member.

13. The surgical obturator according to any one of the preceding claims, and including a protective shield coaxially mounted about the blade member, the protective shield and the blade member being adapted for relative longitudinal movement between a first armed position of the blade member and a second disarmed position of the blade member.

14. The surgical obturator according to claim 13, wherein the protective shield is mounted for longitudinal movement relative to the obturator member.

15. The surgical obturator according to claim 13 or 14, wherein the protective shield is normally biased to a position corresponding to the second disarmed position of the blade member.

16. A surgical obturator, which comprises:
an obturator member defining a longitudinal axis and having proximal and distal ends; and
a generally planar blade member disposed adjacent the distal end of the obturator member, the blade member including peripherally disposed opposed pairs of first and second generally concave surfaces intersecting to define opposed peripheral cutting edges, the peripheral cutting edges extending toward a penetrating end of the blade member, each cutting edge extending in general oblique relation to the longitudinal axis.

17. The surgical obturator according to claim 16, further including:
an obturator housing mounted adjacent the proximal end of the obturator member; and
a protective sleeve coaxially mounted about the obturator member, the protective sleeve adapted for reciprocal longitudinal movement between an armed position of the blade member and a disarmed position of the blade member, the protective sleeve being normally biased to a position corresponding to the disarmed position of the blade member.

18. The surgical obturator according to claim 16 or 17, wherein the cutting edges are generally linear.

19. The surgical obturator according to claim 16 or 17, wherein the cutting edges are generally arcuate.
